# EUROPEAN PATENT SPECIFICATION

(11) **EP 4 162 965 B1**
(45) Date of publication and mention of the grant of the patent: **31.12.2025**
(21) Application number: 21201269.4
(22) Date of filing: 06.10.2021
(51) Int. Cl.: A61M 5/31, A61M 5/44, A61M 5/315, A61B 17/00

(54) **A SYRINGE FOR THE APPLICATION OF A SURGICAL MATERIAL TO A HUMAN OR ANIMAL TISSUE**
SPRITZE ZUR APPLIKATION EINES CHIRURGISCHEN MATERIALS AUF EIN MENSCHLICHES ODER TIERISCHES GEWEBE
SERINGUE POUR L'APPLICATION D'UN MATÉRIAU CHIRURGICAL À UN TISSU HUMAIN OU ANIMAL

(43) Date of publication of application: 12.04.2023
(73) Proprietor: Flowax MD Limited, Dublin 16 (IE)
(72) Inventor: CHOLEVAS, Christos, 42131 Trikala (GR)
(74) Representative: Hoefer & Partner Patentanwälte mbB

(56) References cited:
- US-A1- 2010 106 089
- US-B1- 11 040 144
- US-B2- 9 764 099

## Description

The present invention refers to a syringe for the application of a surgical material to a human or animal tissue. In particular, the present invention refers to a syringe for the application of bone wax to a human or animal bone tissue.

A syringe for the application of haemostatic wax to an application point is known from GR 1008575 A1. The syringe is coated on its inner wall with a metal coating having a projection. The projection of the metal coating is connected to a cable of a diathermy for heating the haemostatic wax inside the syringe.

US 2010/106089 A1 discloses a dispensing assembly that has a dispensing chamber housing, a temperature control device, a thermal sensor, and an interface. The dispensing chamber housing has an inner surface, an outer surface, and a wall thickness. The inner surface partially defines a dispensing chamber for receiving a quantity of a substance. The temperature control device at least partially surrounds the dispensing chamber housing. The temperature control device alters a temperature of a substance in the dispensing chamber. The temperature control device and the thermal sensor are located on a substrate. The substrate is wrapped around an exterior surface of the dispensing chamber housing. The interface is connected to the temperature control device and the thermal sensor. An interface connector is connected to the interface. The distance between the temperature control device and the thermal sensor is approximately equal to the wall thickness of the dispensing chamber housing.

US 9 764 099 B2 discloses syringes having temperature modulation capabilities particularly with regard to changing the temperature of the material being ejected from or injected into the syringe and methods of use. The syringe may comprise a phase transition composition which is to be maintained in one phase within the syringe chamber and ejected in a different phase from the syringe.

It is an object underlying the present invention to provide an improved syringe for the application of a surgical material to a human or animal tissue, in particular of bone wax to a human or animal bone tissue.

The solution to said object is achieved by the features of the independent claim. The dependent claims contain advantageous embodiments of the invention.

A syringe for the application of a surgical material to a human or animal tissue, in particular of bone wax to a human or animal bone tissue, comprises a hollow main body, a pushing element and a heat-generating device for heating the surgical material. The hollow main body has a material receiving space for receiving a surgical material and an outlet. The pushing element is movably arranged in the hollow main body for pushing the surgical material through the outlet. The heat-generating device comprises at least one heat-generating element that is arranged on an outer surface of the hollow main body. This means that the heat-generating device, in particular the at least one heat-generating element, is an integral part of the syringe or in other words integrated in the syringe. The arrangement of the at least one heat-generating element on an outer surface of the hollow main body of the syringe has the advantage that the at least one heat-generating element will not be in contact with a surgical material, when the surgical material is arranged inside the material receiving space. Thus, it is easier to maintain the sterility of the surgical material, as it comes only in contact with the hollow main body and the pushing element during use of the syringe. In other words, there is no (direct) contact between the heat-generating element and the surgical material. In addition, the at least one heat-generating element can be protected by preventing the formation of potential deposits of the surgical material on the at least one heat-generating element that could otherwise be formed in case of a contact between the at least one heat-generating element and the surgical material. Further, the surgical material can be easily pushed out of the hollow main body through the outlet by moving the pushing element, without the latter being obstructed by the at least one heat-generating element. It is noted that the heat-generating device is configured to modify, by generating heat, the physical properties, in particular the viscosity, of the surgical material used, which in turn has a positive effect on its application and adherence on a human or animal tissue. This is particularly advantageous when the surgical material is bone wax, as the modification of the physical properties of bone wax by the generated heat allows the bone wax to be more easily attached to the porous bone surfaces and promote haemostasis in the bone.

The term "hollow main body" means that the main body has an inner space. In particular, the material receiving space corresponds to at least a part of the inner space of the hollow main body. The material receiving space can also be characterized as a material receiving chamber within the framework of the present invention.

It is noted that the arrangement of the at least one heat-generating element on an outer surface of the hollow main body advantageously means that the at least one heat-generating element is completely arranged outside the inner space of the hollow main body.

The heat-generating device is configured to generate heat when provided with electrical energy from an electrical energy source. The electrical energy source is advantageously part of the syringe, in other words integrated in the syringe, and connected to the heat-generating device. The electrical energy source is advantageously a battery.

It is however also possible that the electrical energy source is not part of the heat-generating device. In this case, the syringe is provided with a connection means for connecting an external electrical energy source to the heat-generating device.

The pushing element is advantageously linearly movably arranged inside the hollow main body, in particular inside its inner space. In other words, the pushing element can be pulled and pushed along the hollow main body, in particular along its inner space. Further, the pushing element is advantageously configured to achieve an airtight fit between the hollow main body and the pushing element. To this end, the pushing element preferably comprises a rubber head.

The material receiving space has a volume that varies with the position of the pushing element inside the hollow main body. More particularly, in a first end position of the pushing element, the material receiving space has a maximum volume, whereas in a second end position of the pushing element, the material receiving space has a minimum volume. The minimum volume can be zero. In this case, in its second end position, the pushing element has a maximum possible displacement from the first end position. The first end position can also be called a proximal end position, while the second end position can also be called a distal end position. Preferably, the material receiving space has a maximum volume of 20 ml.

Preferably, the syringe comprises a stopping means for preventing the pushing element from being removed from, in particular pulled out of, the hollow main body. Preferably, the stopping means comprises a projection on the hollow main body and a projection on the pushing element. The projection on the hollow main body is engageable with the projection on the pushing element in the first end position of the pushing element. Thus, an accidental removal of the pushing element out of the hollow main body can be prevented.

The hollow main body is preferably cylindrical. "Cylindrical" may also refer to shapes that slightly deviate from the cylindrical form.

The pushing element can in particular be understood as a plunger. The pushing element is a reciprocating element. The hollow main body can in particular be understood as a barrel.

The surgical material can be any biocompatible material that can be applied to a human or animal tissue and in particular has a temperature-dependent viscosity. The surgical material is preferably a haemostatic wax, in particular bone wax.

The at least one heat-generating element is arranged directly on an outer surface of the hollow main body. "Directly" more particularly means that there is no other element/component arranged between the outer surface of the hollow main body and the at least one heat-generating element in a direction from the at least one heat-generating element to the material receiving space.

The outer surface of the hollow main body, on which the at least one heat-generating element is arranged, is preferably a peripheral surface of the hollow main body. The peripheral surface can also be understood as a circumferential surface.

The at least one heat-generating element is advantageously arranged on a portion of the outer surface of the hollow main body that defines/surrounds the material receiving space.

The at least one heat-generating element is preferably arranged in a recess of the hollow main body formed on the outer surface of the hollow main body. Preferably, the at least one heat-generating element has a complementary shape to the shape of the recess. Thus, a secure arrangement of the at least one heat-generating element on the outer surface of the hollow main body can be achieved.

Preferably, the heat-generating device is configured such that the heat-generating device is heatable up to a predetermined upper temperature limit. Said predetermined upper temperature limit is preferably 80 degrees Celsius.

Preferably, the at least one heat-generating element can be formed as a thermistor, in particular a PTC-thermistor, or a (common) resistor. In case of two heat-generating elements, a combination of thermistors and resistors

A thermistor is a temperature-dependent resistor. A PTC-thermistor is a temperature-dependent resistor with a high positive temperature coefficient (PTC). It exhibits relatively low resistance values at room temperature. When a current flows through a PTC-thermistor, the heat generated raises the temperature of the PTC and thus also its resistance. A resistor is a passive electrical component that implements electrical resistance as a circuit element and consumes electric energy that is converted to heat. A common resistor is not understood as a temperature resistor within the context of the present invention.

In case the at least one heat-generating element is a (common) resistor, the heat-generating device preferably comprises a controlling means that is configured to control such that the heat-generating device is heatable up to a predetermined upper temperature limit.

According to an advantageous embodiment, the heat-generating device comprises a plurality of heat-generating elements. The heat-generating elements may advantageously be arranged on the outer surface of the main hollow body in a longitudinal direction and/or in a circumferential direction of the hollow main body. The longitudinal direction and/or circumferential direction of the hollow main body preferably corresponds/correspond to a longitudinal direction and/or a circumferential direction of the syringe, respectively.

According to an advantageous embodiment of the present invention, the heat-generating elements are arranged on the outer surface of the hollow main body in a non-uniform manner in/with respect to a longitudinal direction of the hollow main body. This particularly means that a distance between two neighbouring heat-generating elements in the longitudinal direction of the hollow main body is not constant for all the heat-generating elements.

Preferably, a density of the heat-generating elements in the longitudinal direction of the hollow main body increases towards a distal end of the material receiving space. This is advantageous, as it can thereby be achieved that heat is transferred to the surgical material as close as possible to the distal end of the material receiving space and to the outlet of the syringe.

The heat-generating device and/or a wall of the hollow main body is/are preferably configured such that a temperature of equal to or higher than 25 degrees Celsius, preferably of equal to or higher than 30 degrees Celsius, is achievable by the heat-generating device in the material receiving space. The heat-generating device and/or a wall of the hollow main body is/are preferably configured such that the temperature achieved in the material receiving space advantageously has an upper limit, which is in particular 40 degrees Celsius.

Preferably, the wall of the hollow main body is configured such that a temperature of equal to or higher than 25 degrees Celsius, preferably of equal to or higher than 30 degrees Celsius, is achievable by the heat-generating device in the material receiving space, when the heat-generating device is heatable to the previously mentioned upper temperature limit of 80 degrees Celsius.

Preferably, the aforementioned temperature is achieved in less than 8 minutes and more preferably in less than 4 minutes. Preferably, the aforementioned temperature is maintained for at least 20 minutes.

Preferably, the wall of the hollow main body is made of plastic, in particular of a plastic compound in order to achieve a homogenous heating of the material receiving space.

The pushing element has a pushing element body.

The pushing element, in particular the pushing element body, may preferably be made of acrylonitrile butadiene styrene (ABS).

A thermal conductivity of the hollow main body is preferably larger than a thermal conductivity of the pushing element, in particular the pushing element body. This facilitates that more heat is transferred to the material receiving space than to the pushing element body.

The thermal conductivity of the hollow main body may preferably lie between 1 W/(m·K) and 10 W/(m·K), more preferably between 5 W/(m·K) and 10 W/(m·K). Thus, heat can be easily transferred to the material receiving space, in particular to the surgical material when placed in the material receiving space.

The pushing element, in particular the pushing element body, may preferably have a thermal conductivity that lies between 0.1 W/(m·K) and 0.3 W/(m·K). Thus, heat loss via the pushing element, in particular the pushing element body, to the outer environment can be reduced or prevented.

Preferably, the wall of the hollow main body, at least in the area where the heat-generating device is arranged, has a thickness between 5 mm and 6 mm, in particular 4 mm.

The heat generating device and/or a wall of the hollow main body is/are configured such that the surgical material is heated by the heat-generating device such that a dynamic viscosity of the surgical material is reduced to equal to or lower than 12 Pa·s, in particular between 1.5 Pa·s and 12 Pa·s. It is understood that the surgical material has a dynamic viscosity higher than 12 Pa·s before being heated.

The pushing element comprises a hollow pushing element body, in which a battery compartment is formed for receiving a battery that is electrically connectable with the heat-generating device. In other words, the hollow pushing element body functions as a battery housing. As there is no external electrical energy source needed to provide the heat-generating device with the required electrical energy and the battery compartment is not formed outside the hollow main body in the syringe according to this configuration, in particular the battery compartment is not provided on an outer surface of the hollow main body, the syringe can be made compact and is easy to handle.

Preferably, the syringe comprises an electrical sliding contact arrangement having a main body contact and a pushing element contact, through which the battery is electrically connectable with the heat-generating device.

In particular, the main body contact may partly be provided inside/integrated in the wall of the main body and partly outside the wall, preferably arranged on an inner side of the wall. The pushing element contact is preferably arranged in a recess formed in a circumferential surface of the pushing element body.

Preferably, the pushing element comprises a pushing element cap, which is separably (removably), in particular threadably, connected to a proximal end of the hollow pushing element body. The pushing element cap can therefore be separated (removed) from the hollow pushing element body and thus provide access to its inner space. In particular, when a battery compartment is formed inside the hollow pushing element, the battery can be inserted into the hollow pushing element by easily removing the pushing element cap. It is understood that in the case of a threadable connection between the hollow pushing element body and the pushing element cap, both the pushing element cap and the hollow pushing element are provided with corresponding threads. The pushing element cap can also be characterized as a proximal cap within the scope of the present invention.

A thermal conductivity of the pushing element cap is preferably smaller than the thermal conductivity of the hollow main body. The pushing element cap may preferably have a thermal conductivity that lies between 0.1 W/(m·K) and 0.3 W/(m·K). Thus, heat loss via the pushing element cap to the outer environment can be reduced or prevented.

The pushing element cap may preferably be made of acrylonitrile butadiene styrene (ABS).

Preferably, the syringe further comprises a distal cap, which is separably (removably), in particular threadably, connected to the hollow main body. The outlet of the syringe is particularly formed in the distal cap. The separable distal cap provides access to the material receiving space. Thus, the surgical material can be inserted in the material receiving space simply by removing the distal cap. It is understood that in the case of a threadable connection between the hollow main body and the distal cap, both the distal cap and the hollow main body are provided with corresponding threads.

The distal cap preferably comprises a tip, in which the outlet is formed. If no distal cap is provided in the syringe, the hollow main body preferably comprises a tip.

Preferably, the tip has a length of 10 mm or higher.

Preferably, the distal cap comprises a luer adapter, in particular a luer lock adapter, for receiving a needle. The luer adapter or also called a luer taper is a standardised system in order to make leak-free connections between two parts. The use of such an adapter is advantageous, as the surgical material can be applied to the tissue without leaks by achieving a leak-free connection between the syringe and the needle.

Preferably, the heat-generating device further comprises at least one heat-generating element that is configured to generate heat and is arranged on an outer surface of the distal cap. Thus, heat can be transferred to the surgical material when exiting through the outlet in the distal cap. This can be advantageous, as in a case that the syringe is put aside during a surgical procedure before having applied all the necessary amount of surgical material onto the human or animal tissue, the surgical material could "solidify" inside the distal cap.

The outer surface of the distal cap is more particularly a peripheral surface of the distal cap.

It is further preferred that the heat-generating device comprises a plurality of heat-generating elements arranged on the outer surface of the distal cap in a circumferential direction of the distal cap, in particular over the whole circumference of the distal cap.

If one or more heat-generating elements are provided on the outer surface of the distal cap, the distal cap preferably has a thermal conductivity between 1 W/(m·K) and 10 W/(m·K), more preferably between 5 W/(m·K) and 10 W/(m·K), thereby facilitating heat transfer between the heat generating element(s) and the material receiving space.

If no heating-generating element is provided on the outer surface of the distal cap, the distal cap preferably has a thermal conductivity smaller than the thermal conductivity of the hollow main body. Thus, heat loss via the distal cap to the outer environment can be reduced or prevented. In this case, the distal cap preferably has a thermal conductivity between 0.1 W/(m·K) and 0.3 W/(m·K). In particular, the distal cap may have the same thermal conductivity with the pushing element, in particular the pushing element body.

Preferably, the material receiving space has a complementary shape to a distal end of the pushing element, in particular a shape of a cylinder or a parallelepiped. Thus, the surgical material can be pushed out of the outlet of the syringe in a more controlled way.

A further aspect of the present invention refers to a syringe kit that comprises a syringe according to the preceding description and a needle.

When the needle is attached to the syringe, the syringe and the needle form a syringe assembly.

These and further details, advantages and features of the present invention will be described based on embodiments of the invention and by taking reference to the accompanying figures. Identical or equivalent elements and elements which act identically or equivalently are denoted with the same reference signs. Not in each case of their occurrence a detailed description of the elements and components is repeated.
- Figure 1: shows a schematic perspective view of a syringe according to a first embodiment of the present invention.
- Figure 2: shows a schematic right view of the syringe of figure 1 when seen from its distal end.
- Figure 3: shows a schematic cross-sectional view of the syringe of figure 2 along the line A-A.
- Figure 4: shows a schematic perspective view of a syringe assembly comprising the syringe of figure 1 and a needle attached to it.
- Figure 5: shows a schematic exploded front view of the syringe assembly of figure 4.
- Figure 6: shows a schematic cross-sectional view of a syringe according to a second embodiment of the present invention.
- Figure 7: shows a schematic cross-sectional view of a syringe according to a third embodiment of the present invention.
- Figure 8: shows a simplified schematic perspective view of a part of a syringe for presenting a feature that can be implemented in the syringe of the first, second or third embodiment,
- Figure 9: shows the part of the syringe of figure 8 in a first position.
- Figure 10: shows the part of the syringe of figure 8 in a second position.

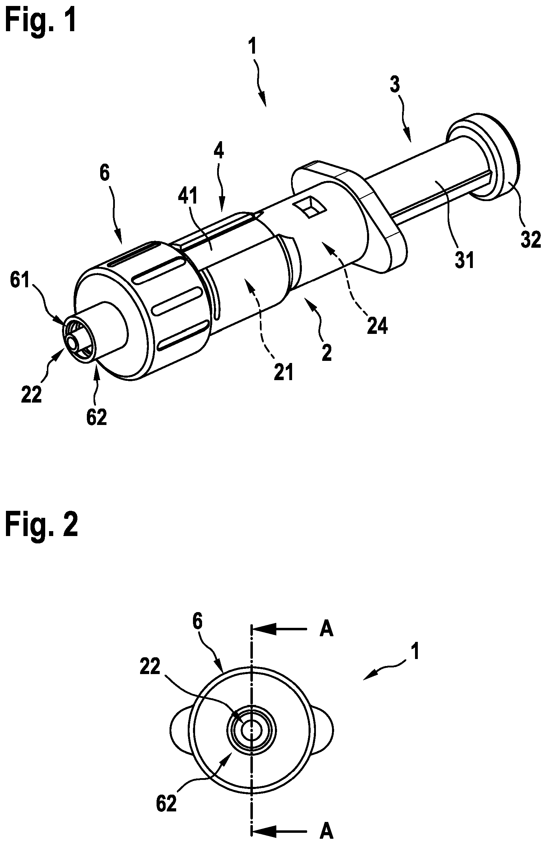

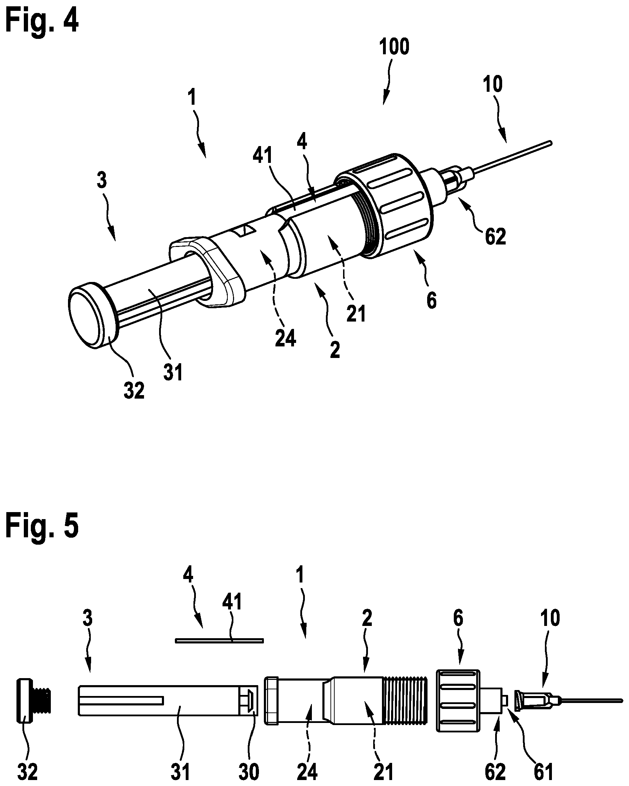

In the following, a syringe 1 for the application of a surgical material to a human or animal tissue and a syringe assembly 100 comprising the syringe 1 and a needle 10 according to a first embodiment of the present invention are described in detail with reference to figures 1 to 5.

The syringe 1 is particularly configured for the application of bone wax (surgical material) to a human or animal bone tissue. Bone wax is a waxy substance used to help mechanically control bleeding from bone surfaces during surgical procedures. Bone wax is a haemostatic wax.

As can be seen from the figures, the syringe 1 comprises a hollow main body 2, a pushing element 3 and a heat-generating device 4 for heating the bone wax.

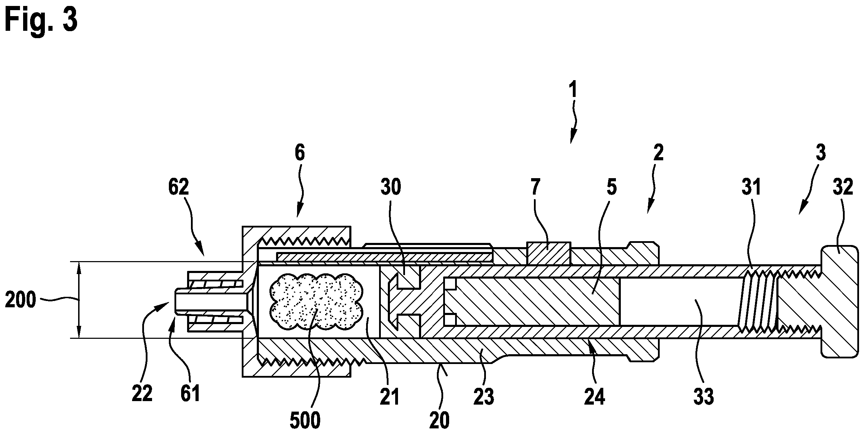

The hollow main body 2 comprises a material receiving space 21 for receiving a piece of bone wax 500 (figure 3), and an outlet 22. The material receiving space 21, which can also be understood as a material receiving chamber, corresponds at least to a part of an inner space 24 of the hollow main body 2, more clearly shown in figure 3. The inner space 24 of the hollow main body 2 is surrounded by a wall 23 of the hollow main body 2.

The hollow main body 2 is preferably cylindrical. This means in particular that the inner space 24 of the hollow main body 2 has a cylindrical shape. Similarly, the material receiving space 21 is also cylindrically formed.

The hollow main body 2 is made of a material that has a thermal conductivity between 1 W/(m·K) and 10 W/(m·K), preferably between 5 W/(m·K) and 10 W/(m·K). In particular, the hollow main body 2 is made of plastic, in particular a plastic compound. Thus, the hollow main body 2 can be easily manufactured and at the same time promote, due to its aforementioned thermal conductivity, heat transfer from the heat-generating device 4 to the material receiving space 21.

The pushing element 3 is linearly movably arranged in the inner space 24 of the hollow main body 2 for pushing the bone wax 500 through the outlet 22. In other words, the pushing element 3 can be moved back and forth inside the inner space 24 of the hollow main body 2.

It is understood that the material receiving space 21 has a volume that varies with the position of the pushing element 3 inside the hollow main body 2. More particularly, in a first end position of the pushing element 3, shown in figures 1, 3 and 4, the material receiving space 21 has a maximum volume, whereas in a second end position of the pushing element 3, the material receiving space 21 has a minimum volume. In its second end position, the pushing element 3 has a maximum possible displacement measured from its first end position. The first end position of the pushing element 3 corresponds to a proximal end position, while the second end position of the pushing element 3 corresponds to a distal end position. The first end position of the pushing element 3 is shown in figures 1, 3 and 4.

Preferably, the material receiving space 21 has a maximum volume of 20 ml. Thus, an adequate quantity of bone wax can be loaded into the material receiving space 21 and cover the needs during the surgical procedure, without increasing the size of the syringe 1. A diameter 200 of the material receiving space 21 (figure 3) preferably lies between 10 mm and 14 mm, in particular equals to 12 mm. The wall 23 of the hollow main body 2 has a thickness between 5 mm and 6 mm, in particular 4 mm.

In order to provide a fixed first end position and prevent an accidental removal of the pushing element 3 from the hollow main body 2, a stopping means may be provided in the syringe 1. Preferably, the stopping means comprises a projection formed on the wall 23 of the hollow main body 2 and a projection formed on the pushing element 3. These two projections engage with each other in the first end position of the pushing element 3, thereby stopping/blocking a further movement of the pushing element 3 towards exiting from the hollow main body 2.

As can further be seen from the figures, the pushing element 3 comprises a hollow pushing element body 31 and a removable pushing element cap (distal cap) 32, which is threadably connected to a proximal end of the hollow pushing element body 31. The pushing element cap 32 can therefore be separated from the hollow pushing element body 31 by a user of the syringe 1 and provide access to its inner space 33. The inner space 33 of the hollow pushing element body 31 is shown in figure 3.

Inside the inner space 33 of the hollow pushing element body 31, a battery compartment is formed for housing a battery 5. The battery 5 is electrically connectable with the heat-generating device 4 for providing the heat-generating device 4 with the needed electrical energy for the generation of heat. The syringe 1 is advantageously configured to be operated at a voltage lower than 20 V. In particular, the battery 5 has a nominal voltage of 12 V (A27 battery). Thus, the syringe 1 requires low electrical energy for its operation and is safe for use on a human or an animal. It is noted that the battery 5 can be considered either as integral part of the syringe 1 or as part of a kit comprising the syringe 1.

Further, the pushing element 3 comprises a rubber head 30 that is attached to a distal end of the pushing element body 31 and seals against the wall 23 of the hollow main body 2. Thus, the pushing element 3 provides an airtight fit between the hollow main body 2 and the pushing element 3 and makes sure that the heated bone wax 500 does not escape from the material receiving space 21.

For the generation of heat, the heat-generating device 4 of the syringe 1 of the present embodiment comprises a heat-generating element 41 that is formed as a PTC-thermistor. Due to its design, the PTC-thermistor varies its electrical resistance in relation to its temperature. As the temperature increases so does the resistance of the PTC/thermistor. When the PTC-thermistor reaches a predetermined upper temperature limit, its resistance is so high, that the PTC-thermistor cannot heat up more. In this way, a temperature inside the material receiving space 21 can be controlled such that it does not exceed a predefined upper limit. Thus, even if an electrical energy source of higher electrical power than the battery 5 described above is connected to the heat-generating element 41, the PTC-thermistor will not exceed its predetermined upper temperature limit and thus the temperature inside the material receiving space 21 will also not exceed its predefined temperature upper limit.

In particular, the heat-generating device 4 and the wall 23 of the hollow main body 2 are each preferably configured such that a temperature between 25 degrees Celsius and 40 degrees Celsius, preferably between 30 degrees Celsius and 40 degrees Celsius, is achieved in the material receiving space 21. Further, the heat-generating device 4 and/or the wall 23 of the hollow main body 2 is/are configured such that the bone wax 500 is heated by the heat-generating device 4 such that a dynamic viscosity of the bone wax 500 is reduced to a dynamic viscosity value of between 1.5 Pa·s and 12 Pa·s. Therefore, the bone wax 500 can be heated to a sufficient extent so that it becomes soft/pliable, whereby it can be easily pressed by the pushing element 3 through the outlet 22.

In order to safeguard the sterility of the bone wax 500 and avoid any reaction between the bone wax 500 and the heat-generating device 4, the heat-generating element 41 is completely positioned outside the inner space 24 of the hollow main body 2. Specifically, the heat-generating element 41 is directly arranged on an outer surface 20 of the hollow main body 2. This means that the heat-generating element 41 is in (direct) contact with the outer surface 20 of the wall 23. The outer surface 20 is a peripheral surface of the hollow main body 2.

For achieving a secure arrangement of the heat-generating element 41 on the outer surface 20 of the hollow main body 2, the heat-generating element 41 is advantageously arranged in a recess 25 formed on the outer surface 20. Preferably, the heat-generating element 41 has a complementary shape to the shape of the recess 25.

Further, the syringe 1 is provided with a removable distal cap 6, which is threadably connected to the hollow main body 2. By removing the separable distal cap 6, the bone wax 500 can be easily placed inside the material receiving space 21.

The outlet 22 of the syringe 1 is formed in the distal cap 6 and in particular in a tip 61 of the distal cap 6. The tip 61 has preferably a length of 10 mm or more.

As can further be seen from figures 1 and 3, the distal cap 6 comprises a luer adapter 62, in particular a luer lock adapter, for receiving the needle 10. It becomes apparent when looking at said figures that the tip 61 is part of the luer adapter 62.

In order to limit the transfer of heat via the distal cap 6 and the pushing element 3, in particular the pushing element body 31 and the pushing element cap 32, to the outer environment, i.e. outside the syringe 1, the distal cap 6 and the pushing element 3 have a thermal conductivity smaller than the thermal conductivity of the hollow main body 2. In particular, the distal cap 6 and the pushing element 3, in particular the pushing element body 31 and the pushing element cap 32, have a thermal conductivity that lies between 0.1 W/(m·K) and 0.3 W/(m·K).

When a surgeon performs a surgical procedure, in which bone bleeding is involved, the surgeon can use the syringe 1 of the present invention for applying bone wax 500 on the bleeding bone tissue and thus achieve haemostasis.

To this end, the surgeon has to first remove the distal cap 6 by unscrewing it from the hollow main body 2 of the syringe 1 and place a certain amount of bone wax 500 inside the main body 2. Then, the surgeon may press a button 7 (figure 3) that is electrically connected to the battery 5 and the heat-generating device 4 in order to activate the heat-generating device 4 for heat generation. When the bone wax 500 becomes soft/pliable due to the heat generated by the heat-generating device 4 and transferred to it through the wall 23 of the main body 2, the surgeon pushes the pushing element 3 forward, preferably by applying pressure to the pushing element cap 32, so that the pushing element 3 moves inside the hollow main body 2 and forces the softened bone wax 500 to exit through the outlet 22 and into the needle 10.

It is, however, possible that the syringe 1 can be used as such, namely without the needle 10, for the application of bone wax 500 on a human or animal bone tissue. Nevertheless, the use of the needle 10 is preferred in minimally invasive surgical procedures, so that a more precise application of the bone wax 500 can be achieved.

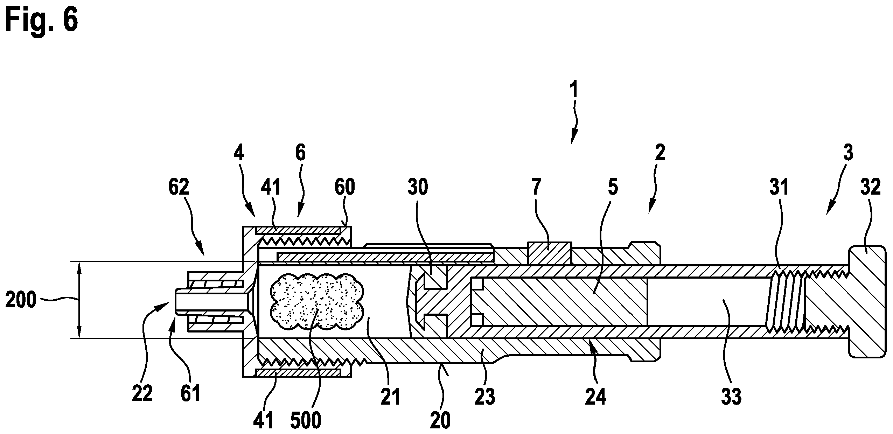

Figure 6 refers to a syringe 1 according to a second embodiment of the present invention.

The syringe 1 according to the second embodiment differs from the syringe 1 according to the first embodiment in that the heat-generating device 4 in the syringe 1 according to the second embodiment further comprises, apart from the heat-generating element 41 on the outer surface 20 of the hollow main body 2, a plurality of heat-generating elements 41 arranged on an outer surface 60 of the distal cap 6. The outer surface 60 of the distal cap 6 is a peripheral surface of the distal cap 6.

More particularly, the heat-generating elements 41 arranged on the outer surface 60 of the distal cap 6, which are preferably also PTC-thermistors like the heat-generating element 41 on the outer surface 20 of the main body 2, are arranged uniformly in a circumferential direction of the distal cap 6 over the whole circumference of the distal cap 6.

For facilitating heat transfer between the heat-generating elements 41 arranged on the outer surface 60 of the distal cap 6, the distal cap 6 has here, contrary to the distal cap 6 of the syringe 1 of the first embodiment, a thermal conductivity that lies between 1 W/(m·K) and 10 W/(m·K), preferably between 5 W/(m·K) and 10 W/(m·K).

Due to the heat-generating elements 41 provided on the outer surface 60 of the distal cap 6, heat can be transferred to the bone wax 500 while it is exiting through the outlet 22 in the distal cap 6.

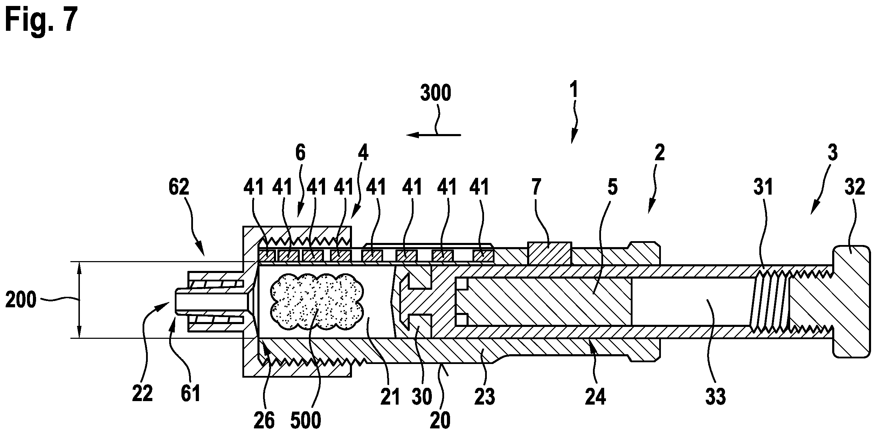

Figure 7 refers to a syringe 1 according to a third embodiment of the present invention.

The syringe 1 according to the third embodiment differs from the syringe 1 according to the first embodiment in that the heat-generating device 4 in the syringe 1 according to the third embodiment comprises a plurality of heat-generating elements 41 arranged on the outer surface 20 of the hollow main body 2 instead of one heat-generating element 41 as it is the case in the syringe 1 according to the first embodiment.

More particularly, the heat-generating elements 41 are arranged on the outer surface 20 of the main hollow body 2 in a non-uniform manner in a longitudinal direction 300 of the hollow main body 2. This particularly means that a distance between two neighbouring heat-generating elements 41 in the longitudinal direction 300 of the hollow main body 2 is not constant for all the heat-generating elements 41.

Advantageously, a density of the heat-generating elements 41 in the longitudinal direction 300 of the hollow main body 2 increases towards a distal end 26 of the material receiving space 21. This is advantageous, as a greater amount of heat can be transferred to the bone wax 500 as close as possible to the distal end 26 of the material receiving space 21 and to the outlet 22 of the syringe 1.

The arrangement of at least one heat-generating element 41 on the outer surface 20 of the hollow main body 2 of the syringe 1 according to the first, second and third embodiment of the present invention has the advantage that the at least one heat-generating element 41 is not in contact with the bone wax 500, when the bone wax 500 is the material receiving space 21. Thus, it is easier to maintain the sterility of the bone wax 500 and prevent it from reacting with the at least one heat-generating element 41. In addition, no bone wax 500 will be deposited on the heat-generating elements 41, thereby achieving an efficient and quick application of the bone wax 500 to the bone tissue.

As the syringe 1 does not require an external electrical energy source for providing the heat-generating device 4 with the required electrical energy and the battery compartment is formed inside the hollow main body 2, the syringe 1 of the present invention has a compact size and is easy to handle. Further, due to its shape, the syringe 1 can be used intuitively by a surgeon for applying the bone wax to a bone tissue during a surgery.

It is further noted that the syringe 1 can in particular be formed as a disposable device.

Though the above presented embodiments of the syringe 1 have been described with reference to surgical procedures related to bone, where bone wax is used to stop bleeding from bone tissues, the syringe 1 of the present invention can also be used with other surgical materials (biocompatible materials), the properties of which, in particular their viscosity, can be influenced/controlled by providing heat to them. Such materials can be used for tissue engineering and drug delivery applications and preferably include thermoresponsive polymer such as poly (N-isopropylacrylamide) (PNIPAM).

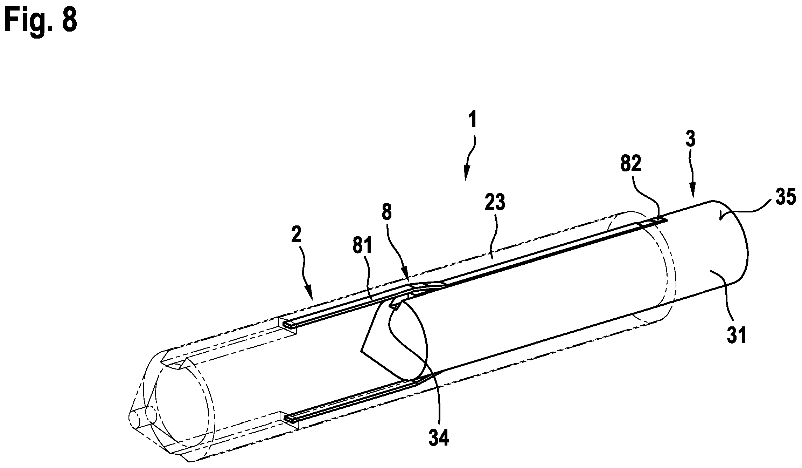

Figures 8 to 10 refer to a feature that can be implemented in any of the syringes 1 of the previously described embodiments of the present invention. For this reason, the syringe 1 in said figures is presented only by a hollow main body 2 and a pushing element 3 in a simplified manner. In order to gain insight to the inner part of the syringe 1, the hollow main body 2 has been drawn in a transparent manner.

As can be seen from the figures, the syringe 1 further comprises an electrical sliding contact arrangement 8. The electrical sliding contact arrangement 8 is configured to act as a switch for electrically connecting/disconnecting the heat-generating device 4 with/from the battery 5.

The electrical sliding contact arrangement 8 comprises a main body contact 81 and a pushing element contact 82 arranged at the main body 2 and the pushing element 3, respectively. The main body contact 81 and the pushing element contact 82 are arranged in such a manner that the main body contact 81 and the pushing element contact 82 can be brought into contact and slide against each other. In particular, the main body contact 81 is partly provided inside/integrated in the wall 23 of the main body 2 and partly outside the wall 23, preferably arranged on an inner side of the wall 23. The pushing element contact 82 is preferably arranged in a recess 34 formed in a circumferential surface 35 of the pushing element body 31.

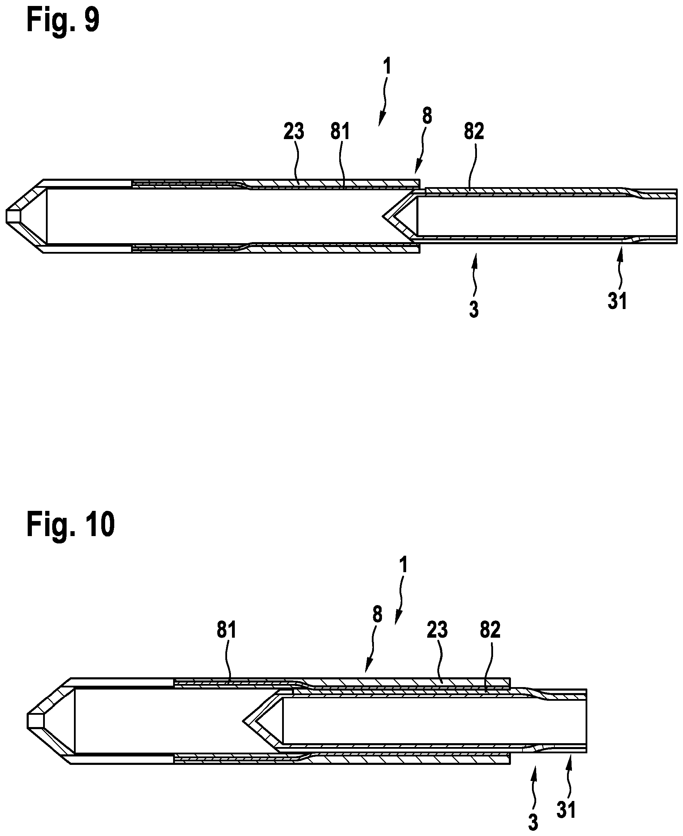

In figures 9 and 10, a first position and a second position of the pushing element 3 are shown, respectively. In the first position, the pushing element 3 is pulled in a proximal direction, whereas in the second position, the pushing element 3 is pushed in a distal direction inside the hollow main body 2. In the first position, the main body contact 81 is not in contact with the pushing element contact 82, so that the heat-generating element(s) 41 is/are not provided with electrical energy from the battery 5. In the second position, the main body contact 81 is in contact with the pushing element contact 82, so that the battery 5 is electrically connected with the heat-generating element(s) 41.

The main body contact 81 and pushing element contact 82 are advantageously formed and arranged such that they are in contact not only in the second position of the pushing element 3 but also through most of the or the whole range of motion of the pushing element 3.

The electrical sliding contact arrangement 8 has the advantage of a compact implementation of the electrical connection between the battery 5 and the heat-generating device 4. The position and movement of the pushing element 3 can further control said electrical connection. An additional advantage of a syringe 1 with such a configuration is the inherent safety feature during the packaging of the syringe 1. In other words, such a configuration enables the syringe 1 to be packaged such that it cannot be activated by chance during transportation, thereby eliminating relevant risks.

The electrical sliding contact arrangement 8 may replace the button 7 or be provided together with the button 7.

The provision of the electrical sliding contact arrangement 8 may be even more advantageous, if the surgical material, in the case of the previously described embodiments bone wax 500, is introduced into the material receiving space 21 from the proximal end of the syringe 1 by removing the pushing element 3, in particular when the syringe 1 does not comprise the removable distal cap 6.

In addition to the foregoing description of the present invention, for an additional disclosure explicit reference is taken to graphic representation of figures 1 to 10.

### List of reference signs

- 1: syringe
- 2: main body
- 3: pushing element
- 4: heat-generating device
- 5: battery
- 6: distal cap
- 7: button
- 8: electrical sliding contact arrangement

- 10: needle
- 20: outer surface
- 21: material receiving space
- 22: outlet
- 23: wall
- 24: inner space
- 25: recess
- 26: distal end
- 30: head
- 31: pushing element body
- 32: pushing element cap
- 33: inner space
- 34: recess
- 35: circumferential surface
- 41: heat-generating element
- 60: outer surface
- 61: tip
- 62: luer adapter
- 81: main body contact
- 82: pushing element contact

- 100: syringe assembly
- 200: diameter
- 300: longitudinal direction
- 500: bone wax

## Claims

1. A syringe (1) for the application of a surgical material to a human or animal tissue, in particular of bone wax (500) to a human or animal bone tissue, comprising:
• a hollow main body (2) with a material receiving space (21) for receiving a surgical material and an outlet (22),
• a pushing element (3), which is movably arranged in the hollow main body (2) for pushing the surgical material through the outlet (22), and
• a heat-generating device (4) for heating the surgical material, wherein the heat-generating device (4) comprises at least one heat-generating element (41) that is arranged on an outer surface (20) of the hollow main body (2),
**characterized in that** the pushing element (3) comprises a hollow pushing element body (31), in which a battery compartment is formed for receiving a battery (5) that is electrically connectable with the heat-generating device (4).

2. The syringe (1) according to claim 1, wherein the at least one heat-generating element (41) is arranged directly on the outer surface (20) of the hollow main body (2), wherein the outer surface (20) of the hollow main body (2) is more particularly a peripheral surface of the hollow main body (2).

3. The syringe (1) according to claim 2, wherein the at least one heat-generating element (41) is arranged in a recess (25) of the hollow main body (2) formed on the outer surface (20) of the hollow main body (2), wherein the heat-generating element (41) preferably has a complementary shape to the shape of the recess (25).

4. The syringe (1) according to any of the preceding claims, wherein the heat-generating device (4) is configured such that the heat-generating device (4) is heatable up to a predetermined upper temperature limit, and/or
wherein at least one heat-generating element (41) is formed as a thermistor, in particular a PTC-thermistor, or a resistor.

5. The syringe (1) according any of the preceding claims, wherein the heat-generating device (4) comprises a plurality of heat-generating elements (41), wherein:
the heat-generating elements (41) are arranged in a longitudinal direction and/or circumferential direction of the hollow main body (2), and/or
the heat-generating elements (41) are arranged on the outer surface (20) of the hollow main body (2) in a non-uniform manner in a longitudinal direction of the hollow main body (2), wherein a density of the heat-generating elements (41) in the longitudinal direction of the hollow main body (2) preferably increases towards a distal end (26) of the material receiving space (21).

6. The syringe (1) according to any of the preceding claims, wherein the heat generating device (4) and a wall (23) of the hollow main body (2) are configured such that a temperature of equal to or higher than 25 degrees Celsius, preferably of equal to or higher than 30 degrees Celsius, and preferably lower than 40 degrees Celsius is achievable by the heat-generating device (4) in the material receiving space (21).

7. The syringe (1) according to any of the preceding claims, wherein the heat generating device (4) and a wall (21) of the hollow main body (2) are configured such that the surgical material is heated by the heat-generating device (4) such that a dynamic viscosity of the surgical material is reduced to equal to or lower than 12 Pa· s, in particular between 1.5 Pa·s and 12 Pa·s.

8. The syringe (1) according to any of the preceding claims, wherein the syringe (1) comprises an electrical sliding contact arrangement (8) having a main body contact (81) and a pushing element contact (82), through which the battery (5) is electrically connectable with the heat-generating device (4).

9. The syringe (1) according to any of the preceding claims, wherein the pushing element (3) comprises a pushing element cap (32), which is separably, in particular threadably, connected to a proximal end of the hollow pushing element body (31).

10. The syringe (1) according to any of the preceding claims, wherein the hollow main body (2) and/or the pushing element (3) is/are made of plastic, and/or
wherein a thermal conductivity of the hollow main body (2) is larger than a thermal conductivity of the pushing element (3),
preferably:
wherein the thermal conductivity of the hollow main body (2) lies between 1 W/(m·K) and 10 W/(m·K), more preferably between 5 W/(m·K) and 10 W/(m·K), and/or
wherein the thermal conductivity of the pushing element (3) lies between 0.1 W/(m·K) and 0.3 W/(m·K).

11. The syringe (1) according to any of the preceding claims, comprising a distal cap (6), which is separably, in particular threadably, connected to the hollow main body (2), wherein the outlet (22) of the syringe (1) is formed in the distal cap (6).

12. The syringe (1) according to claim 11, wherein the distal cap (6) comprises a tip (61), in which the outlet (22) is formed, wherein the tip (61) preferably has a length of 10 mm or more.

13. The syringe (1) according to claim 11 or 12, wherein the distal cap (6) comprises a luer adapter (62), preferably a luer lock adapter, for receiving a needle (10).

14. The syringe (1) according to claim 11 to 13, wherein the heat-generating device (4) further comprises at least one heat-generating element (41) that is configured to generate heat and is arranged on an outer surface (60) of the distal cap (6), wherein the heat-generating device (4) preferably comprises a plurality of heat-generating elements (41) arranged on the outer surface (60) of the distal cap (6) in a circumferential direction of the distal cap (6).

15. The syringe (1) according to any of the preceding claims, wherein the material receiving space (21) has a complementary shape to a distal end of the pushing element (3), in particular a shape of a cylinder or a parallelepiped.

## Patentansprüche

1. Spritze (1) zur Aufbringung eines chirurgischen Materials auf ein menschliches oder tierisches Gewebe, insbesondere von Knochenwachs (500) auf ein menschliches oder tierisches Knochengewebe, umfassend:
• einen hohlen Hauptkörper (2) mit einem Materialaufnahmeraum (21) zum Aufnehmen von einem chirurgischen Material und einem Auslass (22),
• ein Schubelement (3), das im hohlen Hauptkörper (2) zum Schieben des chirurgischen Materials durch den Auslass (22) beweglich angeordnet ist, und
• eine Wärmeerzeugungsvorrichtung (4) zum Erwärmen des chirurgischen Materials, wobei die Wärmeerzeugungsvorrichtung (4) mindestens ein Wärmeerzeugungselement (41) umfasst, das auf einer Außenfläche (20) des hohlen Hauptkörpers (2) angeordnet ist,
**gekennzeichnet dadurch, dass** das Schubelement (3) einen hohlen Schubelementkörper (31) umfasst, in dem ein Batteriefach zum Aufnehmen einer Batterie (5), die elektrisch mit der Wärmeerzeugungsvorrichtung (4) verbindbar ist, ausgebildet ist.

2. Spritze (1) nach Anspruch 1, wobei das mindestens eine Wärmeerzeugungselement (41) direkt auf der Außenfläche (20) des hohlen Hauptkörpers (2) angeordnet ist, wobei die Außenfläche (20) des hohlen Hauptkörpers (2) insbesondere eine Umfangsfläche des hohlen Hauptkörpers (2) ist.

3. Spritze (1) nach Anspruch 2, wobei das mindestens eine Wärmeerzeugungselement (41) in einer Aussparung (25) des hohlen Hauptkörpers (2) angeordnet ist, die auf der Außenfläche (20) des hohlen Hauptkörpers (2) ausgebildet ist, wobei das Wärmeerzeugungselement (41) vorzugsweise eine komplementäre Form zur Form der Aussparung (25) aufweist.

4. Spritze (1) nach einem der vorhergehenden Ansprüche, wobei die Wärmeerzeugungsvorrichtung (4) so konfiguriert ist, dass die Wärmeerzeugungsvorrichtung (4) bis zu einer vorbestimmten oberen Temperaturgrenze erwärmbar ist, und/oder
wobei mindestens ein Wärmeerzeugungselement (41) insbesondere als ein Thermistor, insbesondere als ein PTC-Thermistor, oder als ein Widerstand ausgebildet ist.

5. Spritze (1) nach einem der vorhergehenden Ansprüche, wobei die Wärmeerzeugungsvorrichtung (4) eine Mehrzahl von Wärmeerzeugungselementen (41) umfasst, wobei:
die Wärmeerzeugungselemente (41) in einer Längsrichtung und/oder Umfangsrichtung des hohlen Hauptkörpers (2) angeordnet sind, und/oder
die Wärmeerzeugungselemente (41) auf der Außenfläche (20) des hohlen Hauptkörpers (2) ungleichmäßig in einer Längsrichtung des hohlen Hauptkörpers (2) angeordnet sind, wobei eine Dichte der Wärmeerzeugungselemente (41) in der Längsrichtung des hohlen Hauptkörpers (2) vorzugsweise in Richtung zum distalen Ende (26) des Materialaufnahmeraums (21) zunimmt.

6. Spritze (1) nach einem der vorhergehenden Ansprüche, wobei die Wärmeerzeugungsvorrichtung (4) und eine Wand (23) des hohlen Hauptkörpers (2) so konfiguriert sind, dass durch die Wärmeerzeugungsvorrichtung (4) im Materialaufnahmeraum (21) eine Temperatur von gleich oder größer als 25 Grad Celsius, vorzugsweise von gleich oder größer als 30 Grad Celsius, und vorzugsweise niedriger als 40 Grad Celsius erreichbar ist.

7. Spritze (1) nach einem der vorhergehenden Ansprüche, wobei die Wärmeerzeugungsvorrichtung (4) und eine Wand (21) des hohlen Hauptkörpers (2) so konfiguriert sind, dass das chirurgische Material durch die Wärmeerzeugungsvorrichtung (4) so erwärmt wird, dass eine dynamische Viskosität des chirurgischen Materials auf gleich oder niedriger als 12 Pa·s, insbesondere zwischen 1,5 Pa·s und 12 Pa·s, reduziert wird.

8. Spritze (1) nach einem der vorhergehenden Ansprüche, wobei die Spritze (1) eine elektrische Gleitkontaktanordnung (8) umfasst, die einen Hauptkörperkontakt (81) und einen Schubelementkontakt (82) aufweist, über die die Batterie (5) mit der Wärmeerzeugungsvorrichtung (4) elektrisch verbindbar ist.

9. Spritze (1) nach einem der vorhergehenden Ansprüche, wobei das Schubelement (3) eine Schubelementkappe (32) umfasst, die trennbar, insbesondere verschraubbar, mit einem proximalen Ende des hohlen Schubelementkörpers (31) verbunden ist.

10. Spritze (1) nach einem der vorhergehenden Ansprüche, wobei der hohle Hauptkörper (2) und/oder das Schubelement (3) aus Kunststoff hergestellt ist/sind, und/oder
wobei eine Wärmeleitfähigkeit des hohlen Hauptkörpers (2) größer als eine Wärmeleitfähigkeit des Schubelements (3) ist,
vorzugsweise:
wobei die Wärmeleitfähigkeit des hohlen Hauptkörpers (2) zwischen 1 W/(m·K) und 10 W/(m·K), ferner vorzugsweise zwischen 5 W/(m·K) und 10 W/(m·K), liegt, und/oder
wobei die Wärmeleitfähigkeit des Schubelements (3) zwischen 0,1 W/(m·K) und 0,3 W/(m·K) liegt.

11. Spritze (1) nach einem der vorhergehenden Ansprüche, umfassend eine distale Kappe (6), die trennbar, insbesondere verschraubbar, mit dem hohlen Hauptkörper (2) verbunden ist, wobei der Auslass (22) der Spritze (1) in der distalen Kappe (6) ausgebildet ist.

12. Spritze (1) nach Anspruch 11, wobei die distale Kappe (6) eine Spitze (61) aufweist, in der der Auslass (22) ausgebildet ist, wobei die Spitze (61) vorzugsweise eine Länge von 10 mm oder mehr aufweist.

13. Spritze (1) nach Anspruch 11 oder 12, wobei die distale Kappe (6) einen Luer-Adapter (62), vorzugsweise einen Luer-Lock-Adapter, zum Aufnehmen einer Nadel (10) umfasst.

14. Spritze (1) nach Anspruch 11 bis 13, wobei die Wärmeerzeugungsvorrichtung (4) ferner mindestens ein Wärmeerzeugungselement (41) umfasst, das zur Wärmeerzeugung konfiguriert ist und auf einer Außenfläche (60) der distalen Kappe (6) angeordnet ist, wobei die Wärmeerzeugungsvorrichtung (4) vorzugsweise eine Mehrzahl von Wärmeerzeugungselementen (41) umfasst, die auf der Außenfläche (60) der distalen Kappe (6) in einer Umfangsrichtung der distalen Kappe (6) angeordnet sind.

15. Spritze (1) nach einem der vorhergehenden Ansprüche, wobei der Materialaufnahmeraum (21) eine komplementäre Form zu einem distalen Ende des Schubelements (3), insbesondere eine Form eines Zylinders oder eines Quaders, aufweist.

## Revendications

1. Seringue (1) destinée à l'application d'un matériau chirurgical sur un tissu humain ou animal, en particulier de cire osseuse (500) sur un tissu osseux humain ou animal, comprenant :
• un corps principal creux (2) avec un espace de réception de matériau (21) destiné à recevoir un matériau chirurgical et une sortie (22),
• un élément de poussée (3), qui est agencé de manière mobile dans le corps principal creux (2) pour pousser le matériau chirurgical à travers la sortie (22), et
• un dispositif de génération de chaleur (4) destiné à chauffer le matériau chirurgical, où le dispositif de génération de chaleur (4) comprend au moins un élément de génération de chaleur (41) qui est agencé sur une surface extérieure (20) du corps principal creux (2),
**caractérisée en ce que** l'élément de poussée (3) comprend un corps d'élément de poussée creux (31), dans lequel est formé un compartiment de batterie destiné à recevoir une batterie (5) qui peut être connectée électriquement au dispositif de génération de chaleur (4).

2. Seringue (1) selon la revendication 1, dans laquelle l'au moins un élément de génération de chaleur (41) est agencé directement sur la surface extérieure (20) du corps principal creux (2), dans laquelle la surface extérieure (20) du corps principal creux (2) est plus particulièrement une surface périphérique du corps principal creux (2).

3. Seringue (1) selon la revendication 2, dans laquelle l'au moins un élément de génération de chaleur (41) est agencé dans un évidement (25) du corps principal creux (2) formé sur la surface extérieure (20) du corps principal creux (2), dans laquelle l'élément de génération de chaleur (41) a de préférence une forme complémentaire à la forme de l'évidement (25).

4. Seringue (1) selon l'une quelconque des revendications précédentes, dans laquelle le dispositif de génération de chaleur (4) est configuré de sorte que le dispositif de génération de chaleur (4) peut être chauffé jusqu'à une limite de température supérieure prédéterminée, et/ou
dans laquelle au moins un élément de génération de chaleur (41) est formé comme une thermistance, en particulier une thermistance à coefficient de température positif, PTC, ou une résistance.

5. Seringue (1) selon l'une quelconque des revendications précédentes, dans laquelle le dispositif de génération de chaleur (4) comprend une pluralité d'éléments de génération de chaleur (41), dans laquelle :
les éléments de génération de chaleur (41) sont agencés dans une direction longitudinale et/ou une direction circonférentielle du corps principal creux (2), et/ou
les éléments de génération de chaleur (41) sont agencés sur la surface extérieure (20) du corps principal creux (2) de manière non uniforme dans une direction longitudinale du corps principal creux (2), dans laquelle une densité des éléments de génération de chaleur (41) dans la direction longitudinale du corps principal creux (2) augmente de préférence vers une extrémité distale (26) de l'espace de réception de matériau (21).

6. Seringue (1) selon l'une quelconque des revendications précédentes, dans laquelle le dispositif de génération de chaleur (4) et une paroi (23) du corps principal creux (2) sont configurés de sorte qu'une température égale ou supérieure à 25 degrés Celsius, de préférence égale ou supérieure à 30 degrés Celsius, et de préférence inférieure à 40 degrés Celsius, peut être atteinte par le dispositif de génération de chaleur (4) dans l'espace de réception de matériau (21).

7. Seringue (1) selon l'une quelconque des revendications précédentes, dans laquelle le dispositif de génération de chaleur (4) et une paroi (23) du corps principal creux (2) sont configurés de sorte que le matériau chirurgical est chauffé par le dispositif de génération de chaleur (4) de sorte qu'une viscosité dynamique du matériau chirurgical est réduite à une valeur égale ou inférieure à 12 Pa·s, en particulier à une valeur comprise entre 1,5 Pa·s et 12 Pa·s.

8. Seringue (1) selon l'une quelconque des revendications précédentes, dans laquelle la seringue (1) comprend un agencement à contact glissant électrique (8) ayant un contact de corps principal (81) et un contact d'élément de poussée (82), par l'intermédiaire duquel la batterie (5) peut être connectée électriquement au dispositif de génération de chaleur (4).

9. Seringue (1) selon l'une quelconque des revendications précédentes, dans laquelle l'élément de poussée (3) comprend un capuchon d'élément de poussée (32), qui est relié de manière séparable, en particulier par vissage, à une extrémité proximale du corps d'élément de poussée creux (31).

10. Seringue (1) selon l'une quelconque des revendications précédentes, dans laquelle le corps principal creux (2) et/ou l'élément de poussée (3) sont constitués de plastique, et/ou
dans laquelle une conductivité thermique du corps principal creux (2) est supérieure à une conductivité thermique de l'élément de poussée (3),
de préférence :
dans laquelle la conductivité thermique du corps principal creux (2) est comprise entre 1 W/(m·K) et 10 W/(m·K), plus préférablement entre 5 W/(m·K) et 10 W/(m·K), et/ou
dans laquelle la conductivité thermique de l'élément de poussée (3) est comprise entre 0,1 W/(m·K) et 0,3 W/(m-K).

11. Seringue (1) selon l'une quelconque des revendications précédentes, comprenant un capuchon distal (6) qui est relié de manière séparable, en particulier par vissage, au corps principal creux (2), dans laquelle la sortie (22) de la seringue (1) est formée dans le capuchon distal (6).

12. Seringue (1) selon la revendication 11, dans laquelle le capuchon distal (6) comprend une pointe (61) dans laquelle est formée la sortie (22), la pointe (61) ayant de préférence une longueur de 10 mm ou plus.

13. Seringue (1) selon la revendication 11 ou 12, dans laquelle le capuchon distal (6) comprend un adaptateur Luer (62), de préférence un adaptateur Luer-Lock, destiné à recevoir une aiguille (10).

14. Seringue (1) selon les revendications 11 à 13, dans laquelle le dispositif de génération de chaleur (4) comprend en outre au moins un élément de génération de chaleur (41) qui est configuré pour générer de la chaleur et qui est agencé sur une surface extérieure (60) du capuchon distal (6), dans laquelle le dispositif de génération de chaleur (4) comprend de préférence une pluralité d'éléments de génération de chaleur (41) agencés sur la surface extérieure (60) du capuchon distal (6) dans une direction circonférentielle du capuchon distal (6).

15. Seringue (1) selon l'une quelconque des revendications précédentes, dans laquelle l'espace de réception de matériau (21) a une forme complémentaire à une extrémité distale de l'élément de poussée (3), en particulier une forme de cylindre ou de parallélépipède.
